Europäisches Patentamt

European Patent Office

Office européen des brevets

(19)

(11) Veröffentlichungsnummer: **0 367 982**
**A1**

(12) # EUROPÄISCHE PATENTANMELDUNG

(21) Anmeldenummer: **89118286.7**

(22) Anmeldetag: **03.10.89**

(51) Int. Cl.5: **A61B 17/22**

(30) Priorität: **19.10.88 DE 8813144 U**

(43) Veröffentlichungstag der Anmeldung:
**16.05.90 Patentblatt 90/20**

(84) Benannte Vertragsstaaten:
**AT BE CH DE ES FR GB IT LI NL**

(71) Anmelder: **Günther, Rolf W., Prof. Dr.**
**Brüsseler Ring 73c**
**D-5100 Aachen(DE)**

(72) Erfinder: **Günther, Rolf W., Prof. Dr.**
**Brüsseler Ring 73c**
**D-5100 Aachen(DE)**

(74) Vertreter: **Bauer, Hubert, Dipl.-Ing.**
**Am Keilbusch 4**
**D-5100 Aachen(DE)**

(54) **Vorrichtung zur perkutanen Entfernung von Thromben und Emboli.**

(57) Zur perkutanen Entfernung von Thromben und Emboli aus Venen und Arterien wird ein Embolektomie-Katheter vorgeschlagen, der mit einer Absaugeinrichtung versehen ist. Im Embolektomie-Katheter ist koaxial eine um die Längsachse rotierbare Welle (1) aus einem federelastischen Draht (2) angeordnet. Das distale Ende der Welle (1) ist mit einem Propeller (3) versehen, der innerhalb des Embolektomie-Katheters (4) gelagert ist.

Fig. 2

EP 0 367 982 A1

## Vorrichtung zur perkutanen Entfernung von Thromben und Emboli

Die Erfindung betrifft eine Vorrichtung zur perkutanen Entfernung von Thromben und Emboli aus Venen sowie Arterien und besteht aus einem Embolektomie-Katheter. In diesem ist koaxial ein um seine Längsachse rotierbarer Draht angeordnet. Das distale Ende des Drahtes ist mit einem innerhalb des Katheters gelagerten Rotationskörper bestückt.

Ähnliche Vorrichtungen sind bereits bekannt. Durch Katheter mit einer Vakuumglocke wurde versucht, Lungenemboli durch einfaches Absaugen und Extrahieren zu entfernen. Da diese Technik sehr beschwerlich und zeitaufwendig war, haben sich diese Vorrichtungen nicht allgemein bewährt, obschon bei peripheren Arterien die Absaug-Thrombembolektomie mit zufriedenstellenden Ergebnissen angewendet werden konnte. Allerdings mußten dazu Katheter mit relativ großen Lumenquerschnitten eingesetzt werden.

Zur Ausräumung von Venen bzw. Arterien wurden auch schon Vorrichtungen mit rotierenden Einrichtungen, jedoch ohne Absaugung, in arteriellen angioplastischen Systemen, wie dem Kensey-Katheter, dem Kaltenbach-Katheter und dem atherolytischen Draht oder dem Ritchie-Katheter, verwirklicht. Der erste, 1986 von Ritchie beschriebene Rotationskatheter für die Behandlung von akut thrombotischen Verschlüssen peripherer Arterien war mit einer stumpfen, strahlenundurchlässigen, mit 600 U/min umlaufenden Spitze versehen, mit der eine Auflösung von akuten Thromben möglich war, indem Fibrin um die Welle gewickelt wurde, während die nichtfibrinösen Zellelemente des Gerinnsels in den Kreislauf entlassen wurden. Diese Vorrichtung erwies sich jedoch schon bei 2 bis 8 Tage alten Gerinnseln als unwirksam.

Im Gefolge wurde eine Vorrichtung mit einer umlaufenden Schneidspitze nach Art eines Schleifkopfes entwickelt, der aus Diamanten mit einem Durchmesser von 30 bis 40 $\mu$m bestand. Der Schleifkopf wurde mit 40.000 U/min über einen 1-mm-Führungsdraht vorgeschoben. Im Tierversuch erwies sich diese Vorrichtung auch bei subakuten thrombotischen Verschlüssen als wirksam, allerdings wurden in einem Drittel der so behandelten Arterien Perforationen festgestellt.

Eine Vorrichtung der eingangs beschriebenen Art wurde erstmals 1988 von Beck et al. vorgeschlagen, womit durch einen als Spiralbohrer ausgebildeten Rotationskörper, der innerhalb des Katheters umläuft, eine Zerkleinerung von Teilstücken eines Thrombus oder Embolus herbeigeführt werden soll. Dabei ist auch eine Absaugung der zerkleinerten Teilstücke eines Thrombus oder Embolus als Partikel vorgesehen.

Diese bekannte Vorrichtung hat den Nachteil, daß der Rotationskörper den wesentlichsten Teil des Katheterquerschnitts versperrt und für die Absaugung der Partikel im Bereich des Rotationskörpers nur der Querschnittsteil zur Verfügung steht, den die spiralförmig verlaufenden Nuten im Rotationskörper freilassen. Zudem setzen sich diese naturgemäß sehr engen Nuten im Rotationskörper leicht mit Partikeln zu, wodurch eine wirksame Absaugung insgesamt unterbunden werden kann.

Der Erfindung liegt die Aufgabe zugrunde, eine Vorrichtung der eingangs beschriebenen Art vorzuschlagen, mit der sich die perkutane Entfernung einer großen Anzahl von Thromben und Emboli hauptsächlich im venösen System und den Pulmonalarterien, aber auch die Abreinigung von intravenösen Filtern dadurch verbessern läßt, daß sowohl eine gründliche Zerkleinerung wie auch gleichzeitig eine effektive Absaugung der Verschlußbestandteile während einer kurzen Behandlungsdauer zu gewährleisten ist.

Zur Lösung dieser Aufgabe wird von einer Vorrichtung der im Oberbegriff des Anspruchs 1 genannten gattungsgemäßen Art ausgegangen, welche erfindungsgemäß das im kennzeichnenden Teil desselben angegebene Merkmal aufweist.

Durch den erfindungsgemäßen Propeller, der vorzugsweise aus einem Doppelflügel besteht, ist im Bereich dieses Rotationskörpers der Katheterquerschnitt bis auf den Flügelquerschnitt unversperrt, so daß sich der durch die Absaugeinrichtung im Katheter erzeugte Unterdruck nahezu ungedrosselt über das distale Katheterende hinaus beispielsweise bis auf einen zu entfernenden Thrombus fortsetzt. Von diesem wird daher ein erstes Teilstück in den Katheter hineingesaugt und durch den Propeller zerkleinert, wobei die dabei entstehenden Partikel unmittelbar abgesaugt werden. Unterdessen wird kontinuierlich Teilstück für Teilstück des Thrombus in den Katheter eingesaugt und zerkleinert, bis schließlich auf diese Weise der Thrombus insgesamt abgebaut und seine Bestandteile abgeführt sind.

Nach einer weiteren Ausgestaltung der Erfindung fluchtet die Stirnseite des Propellers mit dem distalen Ende des Katheters.

Durch diese Anordnung erhält nicht nur der Propeller, der vorzugsweise eine S-förmige Gestalt aufweist und zur Verbesserung der Förderleistung auch in axialer Richtung verwunden sein kann, eine sichere Lagerung über seine axiale Erstreckung, sondern der fluchtende Abschluß verhindert auch, daß durch die Rotation des Propellers Beschädigungen an Venen oder Arterien erfolgen. Andererseits reicht die fluchtende Propelleranordnung aus, um jedes auch noch so geringfügig über den Katheteröffnungsquerschnitt eingesaugte Teilstück eines Thrombus oder Embolus durch die Rotation des Propellers zu zerkleinern.

Um die Lagerung des Propellers im Katheter zu optimieren, sieht eine weitere Ausgestaltung der Erfindung vor, daß radiale Außenkanten des Propellers Flächenteile eines Zylinders definieren, dessen Durchmesser geringfügig kleiner ist als der Innendurchmesser des Katheters.

Nach weiteren Ausgestaltungen der Erfindung ist der Propeller mit der Welle, die aus einem elastischen, rostfreien Volldraht oder einem schraubenlinienförmig gewundenen Federdraht besteht, verlötet.

Um den Propeller sicher anzutreiben, ist nach einer weiteren Ausgestaltung der Erfindung die Welle an ihrem proximalen Ende mit der Antriebswelle eines in einem Handgriff eingekapselten Gleichstrommotors gekuppelt und durch einen Simmering abgedichtet.

Der Anschluß einer Absaugpumpe erfolgt nach einer weiteren Ausgestaltung der Erfindung über einen Seiten-Adapter des Katheters.

Schließlich sieht eine Ausgestaltung der Erfindung noch vor, daß der Seiten-Adapter um die Katheter-Längsachse verdrehbar angeschlossen ist.

Durch diese Ausgestaltung läßt sich die Vorrichtung im wesentlichen frei handhaben und anordnen, da mit dem Seiten-Adapter die Absaugeinrichtung insgesamt verschwenkbar ist und somit diese die jeweils günstigste Position einnehmen kann.

In der Zeichnung ist ein Ausführungsbeispiel der erfindungsgemäßen Vorrichtung schematisch dargestellt. Es zeigen:

Fig. 1 eine Welle mit einem Propeller;

Fig. 2 eine Draufsicht auf das distale Ende eines Embolektomie-Katheters und den Propeller in vergrößertem Maßstab;

Fig. 3 einen abgebrochenen Längsschnitt durch die Vorrichtung ohne die Welle mit dem Propeller;

Fig. 4 einen Längsschnitt durch die Vorrichtung gemäß Fig. 3, jedoch mit der Welle und dem Propeller.

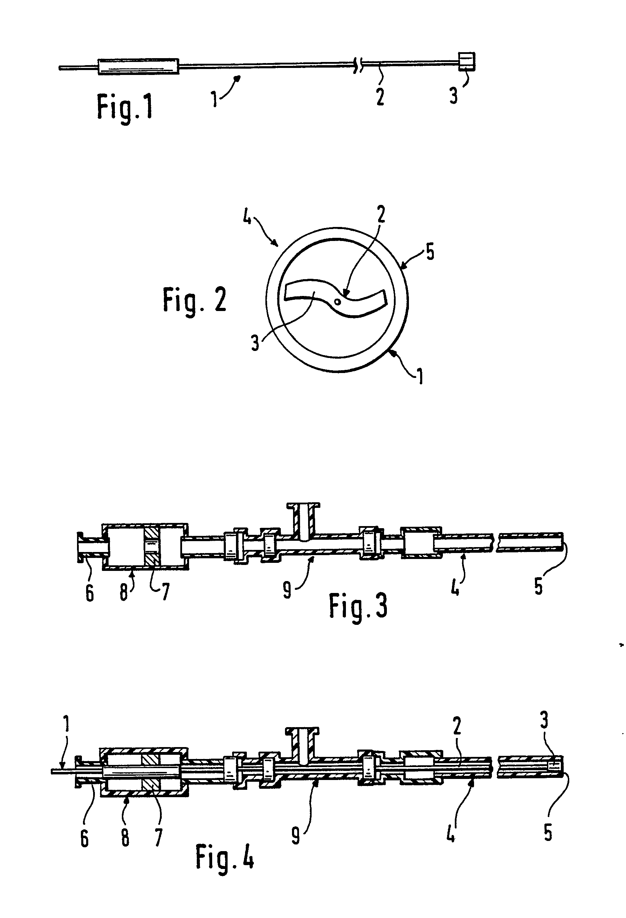

Mit der in Fig. 1 dargestellten Welle 1, die aus einem federelastischen Draht 2 besteht, an dessen einem Ende ein Vitallium-Propeller 3 sitzt, ist der in Fig. 3 dargestellte Teflon-Katheter 4 zu ergänzen. Dazu wird die Welle 1 axial in den Katheter 4 so weit eingeführt, bis die Stirnseite des Propellers 3 mit dem distalen Ende 5 des Katheters 4 fluchtet.

Am proximalen Ende 6 des Katheters 4 ist ein nicht dargestellter 25-V-Gleichstrommotor vorgesehen, dessen Antriebswelle mit der Welle 1 zu kuppeln ist. Durch den wasserdicht in einen Kunststoffhandgriff gekapselten Gleichstrommotor ist die Welle 1 bis 1000 U/min antreibbar, wobei ein Simmering 7 im Kupplungsbereich zwischen dem Katheter 4 und dem Motor einen Austritt von Flüssigkeit rund um die rotierende Welle 2 verhindert.

An einen den Simmering 7 aufnehmenden Kupplungsadapter 8 ist der Katheter 4 mit einem verdrehbaren Seiten-Adapter 9 angeschlossen, über den eine nicht dargestellte Spül- und Absaugeinrichtung am Katheter 4 anschließbar ist. Infolge der Verdrehbarkeit des Katheters 4 in bezug auf den Seiten-Adapter 9 kann während der Embolektomie die Katheterlage verändert werden, ohne die Spül- und Absaugeinrichtung verlagern zu müssen.

Vor dem Beginn des Absaugens wird die Spitze 5 des Katheters 4 direkt in den Thrombus gestoßen. Sodann setzt der Absaugvorgang ein, während der Propeller 3 mit 500-1000 U/min umläuft. Der Thrombus wird folglich fortlaufend in den Katheter 4 eingesaugt und zerkleinert, wobei die Partikel über den Seiten-Adapter 9 abgesaugt werden.

## Ansprüche

1. Vorrichtung zur perkutanen Entfernung von Thromben und Emboli aus Venen und Arterien, bestehend aus einem mit einer Absaugeinrichtung versehenen Embolektomie-Katheter, in dem koaxial eine um die Längsachse rotierbare Welle angeordnet ist, deren distales Ende mit einem innerhalb des Katheters gelagerten Rotationskörper bestückt ist, dadurch gekennzeichnet, daß der Rotationskörper aus einem Propeller (3) besteht.

2. Vorrichtung nach Anspruch 1, dadurch gekennzeichnet, daß der Propeller (3) aus einem Doppelflügel gebildet ist.

3. Vorrichtung nach Anspruch 1 oder 2, dadurch gekennzeichnet, daß die Stirnseite des Propellers (3) mit dem distalen Ende (5) des Katheters (4) fluchtet.

4. Vorrichtung nach mindestens einem der Ansprüche 1 bis 3, dadurch gekennzeichnet, daß der Propeller (3) eine S-förmig gewundene Gestalt aufweist.

5. Vorrichtung nach mindestens einem der Ansprüche 1 bis 4, dadurch gekennzeichnet, daß radiale Außenkanten des Propellers (3) Flächenteile eines Zylinders definieren, dessen Durchmesser geringfügig kleiner ist als der Innendurchmesser des Katheters (4).

6. Vorrichtung nach mindestens einem der Ansprüche 1 bis 5, dadurch gekennzeichnet, daß der Propeller (3) mit der Welle (1), die aus einem elastischen, rostfreien Volldraht (2) oder einem schraubenlinienförmig gewundenen Federdraht besteht, verlötet ist.

7. Vorrichtung nach mindestens einem der Ansprüche 1 bis 6, dadurch gekennzeichnet, daß die Welle (1) an ihrem proximalen Ende mit der Antriebswelle eines in einem Handgriff eingekapselten Gleichstrommotors gekuppelt ist.

8. Vorrichtung nach mindestens einem der Ansprüche 1 bis 7, dadurch gekennzeichnet, daß am Katheter (4) über einen Seiten-Adapter (9) eine Absaugpumpe angeschlossen ist.

9. Vorrichtung nach mindestens einem der Ansprüche 1 bis 8, dadurch gekennzeichnet, daß der Seiten-Adapter um die Längsachse des Katheters (4) verdrehbar angeschlossen ist.

Fig.1

Fig. 2

Fig.3

Fig.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | US-A-4 664 112 (KENSEY et al.) * Spalte 10, Zeilen 21-28; Spalte 11, Zeilen 7-18; Spalte 14, Zeilen 22-24,36-39; Abb. 5,11,14 * | 1,2,4-6 | A 61 B 17/22 |
| Y | | 3,7-9 | |
| | --- | | |
| Y | DE-U-8 707 238 (AESCULAP WERKE AG) * Seite 9; Absatz 3; Abb. 2-4 * | 3 | |
| | --- | | |
| Y | EP-A-0 244 058 (DEVICES FOR VASCULAR INTERV.) * Anspruch 1 * | 7 | |
| | --- | | |
| Y | EP-A-0 177 782 (ADVANCED TECH. LAB. INC.) * Seite 4, Zeilen 12-17; Seite 10, Zeilen 11-15; Abb. 1 * | 8,9 | |
| | --- | | |
| A | DE-A-1 903 618 (NATIONAL RES. DEV. CORP.) * Ansprüche 1,2 * | 3,5 | |
| | --- | | **RECHERCHIERTE SACHGEBIETE (Int. Cl.5)** |
| A | US-A-3 937 222 (BANKO A.) * Spalte 4, Zeilen 6-16; Abb. 9A * | 5 | A 61 B A 61 F |
| | --- | | |
| X,P | WO-A-8 906 517 (STOCKMEIER) * Seite 8, Absatz 3; Abb. 1 * | 1-3 | |
| | ----- | | |

Der vorliegende Recherchenbericht wurde für alle Patentansprüche erstellt

| Recherchenort | Abschlußdatum der Recherche | Prüfer |
|---|---|---|
| DEN HAAG | 11-01-1990 | VILLENEUVE J-M.R.J. |

EPO FORM 1503 03.82 (P0403)

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
..............................................................................
& : Mitglied der gleichen Patentfamilie, übereinstimmendes
    Dokument